(19) Europäisches Patentamt
European Patent Office
Office européen des brevets

(11) Veröffentlichungsnummer: **0 180 081**
**B1**

(12) # EUROPÄISCHE PATENTSCHRIFT

(45) Veröffentlichungstag der Patentschrift: **31.08.88**

(51) Int. Cl.⁴: **B 01 D 19/04**, C 12 N 1/38

(21) Anmeldenummer: **85112971.8**

(22) Anmeldetag: **12.10.85**

(54) **Verwendung von endgruppenverschlossenen Fettalkohol- oder Alkylphenolalkoxylaten zur Schaumbekämpfung in der Zucker- und in der Hefeindustrie.**

(30) Priorität: **19.10.84 DE 3438389**

(43) Veröffentlichungstag der Anmeldung: **07.05.86 Patentblatt 86/19**

(45) Bekanntmachung des Hinweises auf die Patenterteilung: **31.08.88 Patentblatt 88/35**

(84) Benannte Vertragsstaaten: **DE FR GB IT**

(56) Entgegenhaltungen:
**EP-A-0 012 052**
**DE-A-2 164 907**
**DE-A-2 556 544**
**FR-A-1 337 677**
**GB-A-775 483**

(73) Patentinhaber: **BASF Aktiengesellschaft, Carl-Bosch- Strasse 38, D-6700 Ludwigshafen (DE)**

(72) Erfinder: **Dietsche, Wolfram, Dr., Albrecht- Duerer-Ring 34b, D-6710 Frankenthal (DE)**
Erfinder: **Lorenz, Klaus, Dr., Ludwig- Uhland-Strasse 16, D-6520 Worms 1 (DE)**
Erfinder: **Vamvakaris, Christos, Dr., Riedweg 6, D-6701 Kallstadt (DE)**
Erfinder: **Hettche, Albert, Dr., Kleiststrasse 12, D-6717 Hessheim (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 180 081 B1

LIBER, STOCKHOLM 1988

**Beschreibung**

Gegenstand der Erfindung ist die Verwendung von endgruppenverschlossenen Fettalkohol- oder Alkylphenolalkoxylaten zur Schaumbekämpfung in der Zucker- und Hefeindustrie.

Bei der industriellen Verarbeitung zuckerhaltiger Pflanzensäfte, wie es in großem Maßstab bei der Gewinnung von Zucker aus Zuckerrüben und aus Zuckerrohr geschieht, ebenso aber auch bei der Erzeugung von Backhefe unter Verwendung von Melasse, muß durch Zusatz von geeigneten Hilfs mitteln die Schaumbildung auf ein erträgliches Maß herabgesetzt oder ganz verhindert werden.

Verantwortlich für die Bildung beständiger Schäume sind gewisse grenzflächenaktive Stoffe, wie z. B. Huminsäuren, Eiweißsubstanzen, Abbauprodukte von Eiweiß und Stärke, Pektine und Saponine, wie sie in der Natur zahlreich vorkommen. Die Menge der Schaumbildner ist daher je nach Erntejahr und Anbaugebiet verschieden.

Durch die Bildung unerwünschter Schäume kann die Produktion in Zucker- und Hefefabriken stark behindert werden, etwa weil die Kapazität der Anlage nicht ausgenutzt werden kann, oder es können durch die Schaumbildung erhebliche Verluste an Rohsaft bzw. an Hefe eintreten.

Besondere Schwierigkeiten durch eine übermäßige Schaumentwicklung können in einer Zuckerfabrik in den Diffusseuren, in den Klärvorrichtungen sowie in den Carbonisiertanks und in den Verdampfern auftreten. In den Gärbottichen einer Hefefabrik soll sich zwar - wegen der besseren Durchlüftung - eine gewisse Menge Schaum bilden, er darf aber ein bestimmtes Maß nicht überschreiten. Die zur Schaumregulierung eingesetzten Substanzen müssen bei der üblichen Aufarbeitung des Endproduktes, etwa bei der Raffination des Zuckers oder bei der Wäsche der Hefe, weitgehend entfernt werden, so daß sie in dem fertigen Erzeugnis nicht mehr nachgewiesen werden können. Darüber hinaus sollen alle Hilfsmittel, die bei der Herstellung von Nahrungsmitteln verwendet werden, geruch- und geschmacklos und selbstverständlich auch physiologisch völlig unbedenklich sein.

Aus wirtschaftlichen Gründen und um die durch den Zusatz von Entschäumern bedingten artfremden Stoffe in den zuckerhaltigen Substraten möglichst gering zu halten, sind besonders hoch wirksame Entschäumer erwünscht. Bei der Kefeherstellung darf der Entschäumer überdies das Wachstum der Hefezellen und damit die Hefeausbeute nicht beeinträchtigen.

Wichtig für den praktischen Einsatz der Entschäumer ist aber auch ihre lang anhaltende Wirkung. Manche anfänglich stark wirksamen Entschäumer werden nach kurzer Zeit völlig wirkungslos, vermutlich, weil die zunächst schwer löslichen Stoffe allmählich in dem zu entschäumenden Substrat fein dispergiert werden. Dementsprechend müssen in einem solchen Falle ständig weitere Entschäumermengen zugesetzt werden, so daß derartige Hilfsmittel unwirtschaftlich im Verbrauch sind.

Für die Entschäumung in der Zucker- und Hefeindustrie sind seit langem fette Öle, wie Rüböl, Erdnußöl, Olivenöl sowie Wollfett und Mineralöl, im Gebrauch. Auch Fettsäuremonoglyceride, Fettsäurepolyglykolester, Polyalkylenglykole, Ester von Tallölsäuren, Äthylenoxidaddukte an Alkylphosphorsäuren und an verzweigte Fettalkohole sind für diesen Zweck vorgeschlagen worden. Obwohl mit diesen Stoffen zumeist eine mehr oder weniger starke Unterdrückung des Schaumes zu erreichen ist, konnten sie aus verschiedenen Gründen nicht restlos befriedigen, vor allem wegen der erforderlichen zu großen Mengen. Der hohe Verbrauch ist z.T. auf eine nicht genügend lang anhaltende Wirkung zurückzuführen.

In der DE-AS-2 164 907 werden spezielle Schaumbekämpfungsmittel für die Zucker- und Hefeindustrie beschrieben. Die dort beschriebenen Fettalkohol-Alkylenoxidaddukte stellen eine Verbesserung gegenüber dem Stand der Technik dar, ohne die gestellte Aufgabe, nämlich hohe Wirkung bei geringstem Einsatz, voll zu erfüllen. In vielen Fällen muß zur Leistungsverstärkung dann auch auf Kombinationen mit bekannten Schaumdämpfern zurückgegriffen werden.

Aus der DE-A-2 556 544 gehen tert.-Butyletner von alkoxylierten Alkyl- oder Alkenylalkoholen mit 6 bis 22 C-Atomen als schaumarme und ätzalkalibeständige Alkoxylate in Mashinengeschirrspülmitteln insbesondere für die Reinigung von Glas, Geschirr, Flaschen usw. hervor.

Es stellte sich daher die Aufgabe, Schaumdämpfer für die Zucker- und Hefeindustrie zu finden, die bei völliger toxikologischer und physiologischer Unbedenklichkeit in geringsten Mengen angewandt, ein Maxima an Schaumdämpfung liefern.

Überraschenderweise wird diese Aufgabe dadurch gelöst, daß man Alkylenoxid-Anlagerungsprodukte an geradkettige oder verzweigte, gesättigte aliphatische Alkohole mit 6 bis 22 Kohlenstoffatomen oder Alkylphenole mit 6 bis 12 C-Atomen in der Alkylkette, deren Endgruppen durch Veretherung mit Methyl, Ethyl oder Allyl verschlossen sind, als Schaumbekämpfungsmittel in der Zucker- und Hefeindustrie einsetzt. Gegenstand der vorliegenden Erfindung ist die Verwendung von Alkoxylierungsprodukten als Schaumdämpfungsmittel in der Zucker- und Hefeindustrie, dadurch gekkenzeichnet, daß Alkoxylierungsprodukte der Formel I

$$R - O - (X_1)_n - (X_2)_m - (X_3)_p - Z$$

in der

R einen Alkylrest mit 6 bis 22 C-Atomen, einen Alkylphenylrest mit 6 bis 12 C-Atomen im Alkyl,
$X_1$ und $X_3$ Ethylenoxideinheiten, wobei
n und p für eine Zahl von 0 bis 15 stehen und die Summe n + p wenigstens 2 ist,

$X_2$ Propylenoxid- und/oder Butylenoxideinheiten, wobei
m für eine Zahl von 2 bis 15 steht, und
2 Methyl, Ethyl oder Allyl bedeuten, mit einem Trübungspunkt < 75°C verwendet werden.

Durch die speziellen Verbindungen der Formel I wird überraschenderweise eine gegenüber dem Stand der Technik signifikante Verbesserung der Schaumdämpfung erreicht, obwohl dem Fachmann auf dem Tensidgebiet bekannt ist, daß z. B. durch einen Methylendgruppenverschluß meist keine zusätzliche Schaumdämpfung erzielt werden kann. Besonders hervorzuheben gegenüber dem nächsten Stand der Technik, DE-AS-2 164 907, ist die deutliche Verbesserung der Ausgiebigkeit, die durch Endgruppenverschluß erreicht wird. Zum Teil können mit den erfindungsgemäßen Schaumdämpfern die gleiche Wirkung erzielt werden, wie mit der doppelten Menge der nichtverschlossenen OH-gruppenhaltigen Schaumdämpfer.

Bevorzugt sind endgruppenverschlossene Alkoxylierungsprodukte auf Basis von C 8 bis C 18-Fettalkoholen mit 2 bis 10 Ethylenoxid und 2 bis 12 Propylenoxideinheiten, wobei das Verhältnis Ethylenoxid zu Propylenodxis 3 : 1 bis 1 : 3 beträgt und für Z Methyl, Ethyl oder Allyl steht. Ganz besonders bevorzugt sind für R geradkettige oder verzweigte Alkylreste mit 9 bis 15 C-Atomen.

Unter dem Trübungspunkt ist die Temperatur zu verstehen, oberhalb der in einem Gemisch aus Butylendiglykol und Wasser (1 : 3) das verwendete Alkoxylat als Mischung zweier flüssiger Phasen vorliegt (vgl. DIN 53 917).

Ausgangsstoffe für die Herstellung der erfindungsgemäß zu verwendenden Tenside sind definitionsgemäß Fettalkohole oder Fettalkoholgemische mit 6 bis 22 Kohlenstoffatomen. Sie können verzweigt oder geradkettig sein und in Form ihrer Isomerengemische oder ihrer Gemische von der Herstellung her vorliegen.

Es handelt sich hierbei beispielsweise um Octanol, Nonanol, Decanol, Dodecanol, Tetradecanol, Hexadecanol, Octadecanol (Stearylalkohol) sowie deren Gemische.

Technisch besonders bevorzugt sind solche, die gemäß der Ziegler- und insbesondere der Oxosynthese erhalten worden sind. Dabei handelt es sich bevorzugt um Einzelschnitte mit 10 oder 13 oder um Gemische der Alkohole mit 9/11, 13/15 oder 16/18 C-Atomen, nach der Oxosynthese hergestellt. Ebenso günstig sind die nach der Ziegler-Synthese erhaltenen 10/12, 10/14 und 12/16 C-Atomen enthaltenden Alkoholgemische. Besonders vorteilhaft ist der $C_{13}/C_{15}$-Schnitt der nach der Oxosynthese erhaltenen Alkoholfraktion. Auch die $C_{12}/C_{14}$-Kokosfettalkyl- und $C_{16}/C_{18}$-Talgfettalkylreste sind besonders zu nennen.

Von den Alkylphenolen als Ausgangsstoffe seien beispielsweise Hexyl-, Octyl-, Nonyl-, Decyl- oder Dodecylphenol genannt.

Die erfindungsgemäß zu verwendenden Alkoxylate sind teilweise bekannt oder werden in üblicher Weise durch Alkoxylierung hergestellt. Die erhaltenen Alkoxylate werden dann anschließend mit einem Alkylierungsmittel in den entsprechenden Ether übergeführt. Somit ist die Herstellung der erfindungsgemäß zu verwendenden Schaumdämpfer literaturbekannt und bedarf nicht der allgemeinen Beschreibung.

Auf die spezielle Herstellung einiger ausgewählter Verbindungen wird in den Beispielen eingegangen.

Die vorliegenden Beispiele erläutern die Erfindung, ohne sie jedoch zu beschränken.

Herstellungsbeispiele

**Beispiel 1**

In einem Autoklaven werden 20,8 Teile $C_{13}/C_{15}$-Oxoalkohol und 0,1 Teile Kaliumhydroxid vorgelegt. Bei 110 bis 120°C werden kontinuierlich 26,4 Teile Ethylenoxid eingegast. Zur Vervollständigung der Reaktion wird 1 Stunde nachgerührt. Bei 130 bis 140°C werden dann 23,2 Teile Propylenoxid kontinuierlich zugegeben und man läßt 2 Stunden nachreagieren. 19 Teile des erhaltenen Fettalkoholalkoxylates werden bei Raumtemperatur mit der äquimolekularen Menge KOH versetzt und in das Alkoholat übergeführt. Anschließend wird mit 4,45 Teilen Dimethylsulfat verethert. Die anorganischen Reaktionsprodukte werden durch Extraktion mit Wasser vom Endprodukt abgetrennt. Dieser Vorgang wird mehrmals wiederholt, bis eine OH-Zahl des Endprodukts <8 erreicht ist. Der Rest Wasser wird durch Vakuumdestillation entfernt, Salzreste durch Filtration.

Es werden 17 Teile eines Endgruppen-verschlossenen Schaumdämpfers erhalten, dessen OH-Zahl bei 7 liegt. Das Restwasser, bestimmt nach Karl Fischer, liegt bei ca. 0,3 %. Der Trübungspunkt einer 2-%-igen Lösung in Butylendiglykol/Wasser im Verhältnis 1 : 3 liegt bei 68 bis 69°C.

Auf analoge Weise werden die folgenden erfindungsgemäß zu verwendenden Schaumdämpfer der Tabelle 1 hergestellt, entsprechend der Bedeutung von Z wurde mit Dimethylsulfat, Diethylsulfat oder Allylchlorid als Alkylierungsmittel umgesetzt. Die Endgruppenverschlüsse liegen bei einer Rest-OH-Zahl von <15, bevorzugt <10. Die OH-Zahl ist damit ein Maß für die Vollständigkeit der Veretherungsreaktion.

**Tabelle 1**

| Beispiel | R | $(X_1)_n$ | $(X_2)_m$ | $(X_3)_p$ | Z | Trübungspukt °C (2 % in BDG*/Wasser 1:3) |
|---|---|---|---|---|---|---|
| 1 | $C_{13-15}$-Oxoalkyl | $(C_2H_4O)_6$ | $(C_3H_6O)_4$ | - | $CH_3$ | 68-69 |
| 2 | $C_{13-15}$-Oxoalkyl | $(C_2H_4O)_5$ | $(C_3H_6O)_4$ | - | $CH_3$ | 64 |
| 3 | $C_{13-15}$-Oxoalkyl | $(C_2H_4O)_5$ | $(C_3H_6O)_3$ | - | $CH_3$ | 66 |
| 4 | $C_{13-15}$-Oxoalkyl | - | $(C_3H_6O)_4$ | $(C_2H_4O)_2$ | $CH_3$ | 70-71 |
| 5 | $C_{13-15}$-Oxoalkyl | $(C_2H_4O)_6$ | $(C_3H_6O)_4$ | - | Allyl | 64-65 |
| 6 | $C_{16-18}$-Alkyl | $(C_2H_4O)_8$ | $(C_3H_6O)_4$ | - | $CH_3$ | 64 |
| 7 | $C_{10-12}$-Ziegler-Alkyl | $(C_2H_4O)_5$ | $(C_3H_6O)_3$ | - | $CH_3$ | 70-71 |
| 8 | $C_{10-12}$-Ziegler-Alkyl | $(C_2H_4O)_5$ | $(C_3H_6O)_3$ | - | $C_2H_5$ | 68 |
| 9 | $C_{10-12}$-Ziegler-Alkyl | $(C_2H_4O)_5$ | $(C_3H_6O)_3$ | - | Allyl | 69-70 |
| 10 | $C_{13-15}$-Oxoalkyl | $(C_2H_4O)_5$ | $(C_3H_8O)_2$ | - | $CH_3$ | 68 |
| 11 | $C_{13-15}$-Oxoalkyl | $(C_2H_4O)_6$ | $(C_4H_8O)_2$ | - | $CH_3$ | 61 |
| 12 | Octylphenol | $(C_2H_4O)_6$ | $(C_3H_6O)_4$ | - | $CH_3$ | 64-66 |
| 13 | $C_{9-11}$-Oxoalkyl | $(C_2H_4O)_5$ | $(C_3H_6O)_3$ | - | $C_2H_5$ | 69-70 |
| 14 | $C_{22}$-Alkyl | $(C_2H_4O)_8$ | $(C_3H_6O)_3$ | - | $CH_3$ | 62 |
| 15 | $C_8$-Oxoalkyl | - | $(C_3H_6O)_6$ | $(C_2H_4O)_3$ | $CH_3$ | 68-70 |

* Butylendiglykol

Anwendungstechnische Beispiele

Die Wirksamkeit von Entschäumern auf Lösungen, wie sie in der Zucker- und Hefeindustrie vorkommen, kann im Labor in nachfolgender Weise geprüft werden.

In einer kontinuierlich arbeitenden Umlaufapparatur mit einem kalibrierten Rohr von 6 cm Durchmesser strömt ein Prüfstrahl mit konstantem Druck in eine Vorratslösung, die einen Schaumgenerator enthält. Hierbei baut sich eine spezifische Schaumhöhe auf, die in ml gemessen wird und ein Maß für die Schaumbildung ist. Wird der Prüflösung neben dem Schaumgenerator noch ein Schaumdämpfer zugesetzt, kann die Schaumdämpferwirkung ermittelt werden. Als Schaumgenerator werden ein Alkylglucosid oder ein käufliches Saponin in Mischung mit einem $C_{13}/C_{15}$-Oxoalkoholethersulfat verwendet. Entsprechend der Temperatur in den Diffusseuren, in den Klärvorrichtungen oder in den Verdampfern wurden die Prüfungen bei 70 bis 75°C durchgeführt.

Die Ergebnisse sind in den folgenden Tabellen 2 und 3 zusammengestellt. Zur Schaumerzeugnung wird eine Lösung von 0,4 g/l Decylglucosid verwendet und das Schaumvolumen nach 1 bis 5 Minuten in Abhängigkeit von den unterschiedlichen Schaumdämpfern gemessen.

**Tabelle 2**

| Schaumdämpfer | Menge/ppm | Schaumvolumen (ml) 1 | 2 | 3 | 4 | 5 min |
|---|---|---|---|---|---|---|
| ohne Schaumdämpfer | - | 17 | 26 | 32 | 37 | 37 |
| Beispiel 1 | 15 | 6 | 11 | 13 | 14 | 15 |
| Beispiel 10 | 15 | 12 | 18 | 17 | 15 | 15 |
| Beispiel 11 | 15 | 9 | 18 | 20 | 17 | 16 |
| Beispiel 2 | 15 | 6 | 10 | 12 | 14 | 15 |
| Beispiel 4 | 15 | 5 | 10 | 12 | 13 | 14 |
| Beispiel 9 | 15 | 8 | 13 | 15 | 16 | 17 |
| Beispiel 8 | 15 | 9 | 12 | 14 | 16 | 18 |

**Tabelle 3**

Vergleichsbeispiele ohne Endgruppenverschluß (Formel I, Z = H)

| Schaumdämpfer | Menge/ppm | Schaumvolumen (ml) 1 | 2 | 3 | 4 | 5 min |
|---|---|---|---|---|---|---|
| gemäß Beispiel 1 Z = H | 15 | 15 | 21 | 23 | 25 | 25 |
| | 30 | 8 | 11 | 13 | 15 | 15 |
| gemäß Beispiel 10 Z = H | 15 | 14 | 20 | 22 | 23 | 25 |
| gemäß Beispiel 11 Z = H | 15 | 13 | 20 | 22 | 25 | 26 |
| | 30 | 8 | 17 | 19 | 20 | 21 |

Aus den Ergebnissen geht hervor, daß durch die Veretherung in nicht naheliegender Weise eine Verstärkung der schaumdämpfenden Wirkung auftritt. In der Regel muß bei einer nicht endgruppenverschlossenen Verbindung die doppelte Menge, 30 ppm, verwendet werden, um eine einigermaßen entsprechende Schaumdämpfung zu erreichen.

Analoge Ergebnisse werden erhalten, wenn anstelle von Decylglucosid Saponin MT zusammen mit einem $C_{13-15}$-Oxoalkoholethersulfat als Schaumerzeuger eingesetzt wird. Wie in der Tabelle 2 kann auch unter diesen veränderten Bedingungen gezeigt werden, daß beim Übergang von Verbindungen der Formel 1 mit Z = H nach Z = Methyl, Ethyl oder Allyl das Schaumdämpfungspotential in vielen Fällen verdoppelt wird.

Die erfindungsgemäßen Produkte zeichnen sich nicht nur durch eine ausgezeichnete Schaumdämpfung bei den Hochtemperatur-Prozessen in der Zucker- und Hefeindustrie aus. Sie können infolge ihrer Trübungspunkte und zugleich ausgezeichneten Dispergiervermögens auch ähnlich erfolgreich bei der 20°C-Rübenwäsche oder bei der Schaumdämpfung in Gärbottichen eingesetzt werden. Auch hier ist ebenfalls die geringe Einsatzmenge von großem Vorteil.

Neben der Anwendung als Einzelverbindung können die erfindungsgemäßen Schaumdämpfer auch in Kombination mit bekannten nativen Produkten, wie Glycerinmonooleat, Wollfett, Paraffinöl, Bizinusöl, Fettalkohol, oder auch als Verstärker zu handelsüblichen Entschäumern, die sich ihrerseits beispielsweise aus den Komponenten Seife, Fettalkohol und Kohlenwasserstoffen, Erdnußöl, Glycerinmonooleat, und Mineralöl, Vorlauffettsäureethoxylat und Glycerinmonooleat zusammensetzen, verwendet werden.

Auch im Bereich der Hefeindustrie kann mit den erfindungsgemäßen Produkten das Schaumprofil in Gärbottichen gezielt gesteuert werden. Hervorzuheben und somit vorteilhaft sind die geringeren Aufwandmengen gegenüber den bisher verwendeten Produkten.

**Patentansprüche**

1. Verwendung von Alkoxylierungsprodukten als Schaumdämpfungsmittel in der Zucker- und Hefeindustrie, dadurch gekennzeichnet, daß Alkoxylierungsprodukte der Formel I

$$R - O - (X_1)_n - (X_2)_m - (X_3)_p - Z$$

in der

R einen Alkylrest mit 6 bis 22 C-Atomen, einen Alkylphenylrest mit 6 bis 12 C-Atomen im Alkyl,

$X_1$, und $X_3$ Ethylenoxideinheiten, wobei

n und p für eine Zahl von 0 bis 15 stehen und die Summe n + p wenigstens 2 ist, $X_2$ Propylenoxid- und/oder Butylenoxideinheiten, wobei

m für eine Zahl von 2 bis 15 steht, und

Z Methyl, Ethyl oder Allyl bedeuten, mit einem Trübungspunkt < 75°C verwendet werden.

2. Verwendung von Alkoxylierungsprodukten der Formel I nach Anspruch 1, in denen R einen Alkylrest mit 8 bis 18 Kohlenstoffatomen bedeutet.

3. Verwendung von Alkoxylierungsprodukten der Formel I nach Anspruch 1 oder 2, in denen n + p eine Zahl von 2 bis 10 und m eine Zahl von 2 bis 12 bedeuten.

4. Verwendung von Alkoxylierungsprodukten der Formel I nach Anspruch 1 bis 3, in denen das Molverhältnis von Ethylenoxid zu Propylenoxid 3 : 1 bis 1 : 3 beträgt.

5. Verwendung von endgruppenverschlossenen Alkoxylierungsprodukten der Formel I nach den Ansprüchen 1 bis 4, in denen R ein Gemisch von Alkylresten mit $C_{9-11}$-, $C_{13-15}$- oder $C_{10-14}$-C-Atomen bedeutet.

6. Verwendung von endgruppenverschlossenen Alkoxylierungsprodukten gemäß den Ansprüchen 1 bis 5 zusammen mit anderen bekannten Entschäumern.

**Claims**

1. Use of an oxyalkylation product as an antifoam in the sugar industry and the yeast industry, wherein the oxyalkylation product used is of the formula I

$$R - O - (X_1)_n - (X_2)_m - (X_3)_p - Z$$

where
R is alkyl of 6 to 22 carbon atoms or alkylphenyl where alkyl is of 6 to 12 carbon atoms,
$X_1$ and $X_3$ are each ethylene oxide units
n and p are each from 0 to 15 and the sum of n and p is not less than 2, the groups
$X_2$ are propylene oxide and/or butylene oxide units,
m is from 2 to 15 and
Z is methyl, ethyl or allyl, and has a turbidity point of < 75° C.

2. Use of an oxyalkylation product of the formula I as claimed in claim 1, where R is alkyl of 8 to 18 carbon atoms.

3. Use of an oxyalkylation product of the formula I as claimed in claim 1 or 2, where n + p is from 2 to 10 and m is from 2 to 12.

4. Use of an oxyalkylation product of the formula I as claimed in any of claims 1 to 3, in which the molar ratio of ethylene oxide to propylene oxide is from 3 : 1 to 1 : 3.

5. Use of an oxyalkylation product of the formula I as claimed in any of claims 1 to 4, which possesses blocked terminal groups and in which R is a mixture of alkyl radicals of 9 - 11, 13 - 15 and 10 - 14 carbon atoms.

6. Use of an oxyalkylation product as claimed in any of claims 1 to 5 possessing blocked terminal groups together with other conventional antifoams.

**Revendications**

1. Utilisation de produits d'alcoxylation comme additifs antimousse dans l'industrie du sucre et des levures, caractérisée en ce qu'on utilise des produits d'alcoxylation de formule I

$$R - O - (X_1)_n - (X_2)_m - (X_3)_p - Z$$

dans laquelle
R représente un radical alkyle à 6 - 22 atomes de carbone, un radical alkylphényle à 6 - 12 atomes de carbone dans le groupement alkyle,
$X_1$ et $X_3$ représentent des motifs oxyde d'éthyléne,
n et p étant mis chacun pour un nombre de 0 à 15 et la somme n + p étant au moins égale à 2,
$X_2$ représente des motifs oxyde de propylène et/ou oxyde de butyléne,
m étant mis pour un nombre de 2 à 15, et
Z représente un radical méthyle, éthyle ou allyle, ces produits ayant un point de trouble < 75° C.

2. Utilisation de produits d'alcoxylation de formule I selon la revendication 1, dans lesquels R représente un radical alkyle à 8 - 18 atomes de carbone.

3. Utilisation de produits d'alcoxylation de formule I selon la revendication 1 ou 2, dans lesquels n + p est un nombre de 2 à 10 et m est un nombre de 2 à 12.

4. Utilisation de produits d'alcoxylation de formule I selon l'une quelconque des revendications 1 à 3, dans lesquels le rapport molaire de l'oxyde d'éthylène à l'oxyde de propylène est compris entre 3 : 1 et 1 : 3.

5. Utilisation de produite d'alcoxylation à groupement terminal bloqué, de formule I selon l'une quelconque des revendications 1 à 4, dans lesquels R représente un mélange de radicaux alkyle à 9 - 11, 13 - 15 ou 10 - 14 atomes de carbone.

9. Utilisation de produite d'alcoxylation à groupement terminal bloqué, de formule I selon l'une quelconque des revendications 1 à 5, en combinaison avec d'autres agents antimoussants connus.